Europäisches Patentamt

European Patent Office

Office européen des brevets

(19)

(11) EP 0 600 774 B1

(12) FASCICULE DE BREVET EUROPEEN

(45) Date de publication et mention
de la délivrance du brevet:
06.08.1997 Bulletin 1997/32

(51) Int. Cl.⁶: **C07C 265/14**, C08G 18/72
// (C08G18/72, 101:00)

(21) Numéro de dépôt: 93402876.2

(22) Date de dépôt: 29.11.1993

(54) **Mélange de polyisocyanates pour la préparation de mousses souples de polyuréthanne de haute élasticité**

Mischungen von Polyisocyanaten zur Herstellung von weichen hochelastischen Polyurethan-Schaumstoffen

Polyisocyanate mixture for the preparation of flexible highly elastic polyurethane foams

(84) Etats contractants désignés:
AT BE CH DE DK ES FR IE IT LI NL

(30) Priorité: 04.12.1992 FR 9214622

(43) Date de publication de la demande:
08.06.1994 Bulletin 1994/23

(73) Titulaire: RHONE-POULENC CHIMIE
92408 Courbevoie Cédex (FR)

(72) Inventeur: **Parron, Jean-Claude**
**F-69300 Caluire (FR)**

(74) Mandataire: **Dubruc, Philippe et al**
**RHONE-POULENC CHIMIE,**
**Direction de la Propriété Industrielle,**
**25, Quai Paul Doumer**
**92408 Courbevoie Cédex (FR)**

(56) Documents cités:
DE-A- 1 924 302      FR-A- 2 176 926
US-A- 3 832 311

**Description**

La présente invention concerne un mélange de polyisocyanates permettant d'obtenir des mousses souples de polyuréthanne de haute élasticité.

Elle concerne également l'utilisation de ce mélange de polyisocyanates pour la préparation de mousses souples de polyuréthanne de haute élasticité.

Les mousses souples de polyuréthanne sont des matériaux connus dont l'usage est très répandu. Ces mousses sont généralement préparées à partir de polyisocyanates et de polyéther-polyols. Elles peuvent être distinguées, selon leurs propriétés élastiques, en mousses dites "standard" et en mousses de haute élasticité. Ces mousses peuvent comprendre également des additifs, tels que notamment des catalyseurs, des tensio-actifs et des agents d'expansion.

Le toluylène diisocyanate (TDI) est le polyisocyanate utilisé pour la préparation des mousses dites "standard" ou traditionnelles. Le TDI-80, consistant en un mélange isomérique de 80 % massique de l'isomère 2,4 et de 20 % massique de l'isomère 2,6, est le mélange le plus couramment utilisé pour cette préparation. Dans certains cas, on utilise également des mélanges plus riches en isomères 2,6 du toluylène diisocyanate, tels que notamment le mélange correspondant au rapport massique 65/35 des isomères 2,4 et 2,6 du toluylène diisocyanate. Cependant, dans certaines applications de ces mousses, telles que notamment le rembourrage, la matelassure et le coussinage, il leur est souvent reproché un manque de confort. C'est-à-dire qu'une faible variation de la force de compression appliquée à ces mousses s'accompagne d'une forte variation de la déformation. Ce phénomène peut notamment être expliqué par la présence d'un "plateau" sur la courbe de déformation des mousses en fonction de la compression exercée.

Il est maintenant possible de fabriquer des mousses, dites mousses souples de haute élasticité, donnant un confort amélioré. Celles-ci se caractérisent par une courbe de déformation en fonction de la compression appliquée, dont la forme de plateau est fortement diminuée. Elles présentent en outre une cellularisation moins régulière.

Différents procédés pour préparer de telles mousses peuvent être mis en oeuvre.

Ainsi, il est connu, d'une part, de modifier le polyéther-polyol, utilisé habituellement pour la préparation des mousses traditionnelles. Ces modifications peuvent consister à greffer sur le polyéther-polyol au moins un monomère insaturé, choisi notamment parmi le styrène et l'acrylonitrile. On peut aussi mettre en contact les polyéther-polyols avec des charges organiques comme les polyurées.

Il est connu, d'autre part, de modifier le toluylène diisocyanate (TDI modifié) soit en le mélangeant avec du diphényl méthane diisocyanate brut, soit en le remplaçant par du toluylène diisocyanate modifié chimiquement.

Cependant, en mettant en oeuvre ces types de TDI modifié, on obtient des mousses pouvant présenter une déformation rémanente à l'humidité trop élevée, plus particulièrement dans le cas du moulage à froid. La déformation rémanente est mesurée après une compression à 70 % de déflection à 50°C et à 95 % d'humidité relative, selon la norme NF-T56-112.

Par ailleurs, depuis que l'utilisation des chlorofluorocarbones en tant qu'agents d'expansion physique, est bannie de la communauté internationale, il est connu de les remplacer pu des agents d'expansion chimique comme l'eau. Or le fait d'augmenter la quantité d'eau dans le procédé a pour conséquence de rendre les mousses résultantes plus sensibles à la déformation rémanente humide.

On peut également reprocher à ces produits moussés à l'eau uniquement, de manquer de souplesse ou d'avoir un excès de portance. En effet, l'eau favorise la formation, dans la matrice du polymère, de segments rigides du type urée.

Enfin, les mousses obtenues à partir d'un mélange de toluylène diisocyanate et de diphényl méthane diisocyanate brut présentent en outre une tenue médiocre au déchirement.

Il est aussi connu de remplacer le toluylène diisocyanate pu des dérivés du diphényl méthane diisocyanate. Cependant, l'inconvénient d'une telle méthode est qu'il est nécessaire d'utiliser des agents d'expansion physiques pour obtenir des mousses de faible densité.

L'utilisation de mélanges comprenant du toluyène diisocyanate sous forme allophanate, combiné à des isocyanates exempts de telles liaisons, pour donner des mousses ignifuges, est exemplifiée dans le brevet US 3 832 311. Cependant, rien ne permet de conclure que l'utilisation de tels mélanges est appropriée pour l'obtention de mousses souples de haute élasticité.

La présente invention a donc pour objet de proposer un nouveau mélange de polyisccyanates ne présentant pas les inconvénients mentionnés ci-dessus, et d'obtenir des mousses souples de polyuréthanne de haute élasticité dont la déformation rémanente est nettement améliorée.

On a maintenant trouvé que ce but et d'autres sont atteints par la présente invention qui concerne un mélange de polyisocyanates comprenant du toluylène diisocyanate modifié chimiquement et du 1,6-hexaméthylène diisocyanate dont la quantité est comprise entre 0,5 et 30 % en poids dudit mélange d'isocyanates.

L'invention concerne également l'utilisation de ce mélange pour la préparation de mousses souples de polyuréthanne de haute élasticité en mettant en oeuvre le procédé de polyaddition d'isocyanates.

Il a été effectivement constaté que l'utilisation du mélange selon l'invention, permet de préparer des mousses souples de polyuréthanne de haute élasticité présentant les avantages mentionnés précédemment.

Ces mousses sont donc particulièrement bien adaptées pour la réalisation de sièges employés dans les domaines de l'automobile et de l'ameublement.

En outre, l'utilisation du mélange selon l'invention permet d'une façon avantageuse, de conserver la cinétique de moussage et de ne pas augmenter la pression maximale développée lors de la préparation des mousses. Ce dernier avantage est intéressant pour l'obtention de produits moulés, car il permet de travailler avec des moules plus légers et de limiter les pertes de matières premières au niveau des surfaces de jointoiements du moule.

Par ailleurs, les mousses obtenues par mise en oeuvre du mélange selon l'invention présentent avantageusement de faibles densités, sans qu'il soit nécessaire d'utiliser des agents d'expansion physiques.

Le premier objet de l'invention concerne donc un mélange de polyisocyanates comprenant du toluylène diisocyanate modifié chimiquement et du 1,6-hexaméthylène diisocyanate.

Par toluylène diisocyanate modifié chimiquement (ou toluylène diisocyanate modifié), on entend des dérivés du toluylène diisocyanate comprenant des liaisons du type allophanate, biuret, uréthanne, urée, carbodiimide ou isocyanurate.

Ces modifications chimiques sont mises en oeuvre par tout moyen connu de l'homme du métier. Ainsi, pour créer des liaisons de type isocyanurate, il est connu de traiter thermiquement l'isocyanate, en présence, le cas échéant d'un catalyseur. Parmi ceux-ci on peut citer notamment les phosphines, les amines comme la pyridine, ou encore les bases de Mannich. Par ailleurs, l'obtention de liaisons de type carbodiimide peut être réalisée en traitant l'isocyanate avec un catalyseur comme l'oxyde de phospholine. On peut aussi envisager de faire réagir l'isocyanate avec au moins une amine ou de l'eau, pour obtenir des liaisons de type urée ; ces liaisons pouvant elles mêmes être transformées par un traitement thermique, en liaisons de type biuret. Une autre modification possible de l'isocyanate consiste à faire réagir celui-ci avec un glycol ou un polyol de bas poids moléculaire pour donner des liaisons uréthannes ; ces dernières pouvant conduire, par chauffage, à des liaisons de type allophanate. Bien entendu, tous ces exemples ne sont donnés qu'à titre indicatif et ne constituent en aucun cas une liste exhausive des moyens connus que l'on peut mettre en oeuvre.

Tout toluylène diisocyanate utilisé habituellement dans les formulations de moussage, peut être modifié chimiquement pour être ensuite utilisé dans le mélange de la présente invention. Le toluylène diisocyanate de départ correspond, de préférence, à un mélange des isomères 2,4 et 2,6 du toluylène diisocyanate. Plus particulièrement, on utilise le mélange isomérique de 80 % massique de l'isomère 2,4 et de 20 % massique de l'isomère 2,6.

Le toluylène diisocyanate modifié utilisé dans la présente invention est, de préférence, un toluylène diisocyanate biurétisé, c'est-à-dire comprenant des liaisons de type biuret. Il peut être obtenu par tout moyen connu en soi, notamment par réaction du toluylène diisocyanate avec de l'eau ou tout composé, comme des sels hydratés, libérant de l'eau à une température inférieure à celle de destruction de l'isocyanate biurétisé. On peut encore le préparer, à titre d'exemple, par réaction avec un alcool facilement déshydratable et de préférence un alcool tertiaire, avec une amine ou encore avec un acide-alcool facilement lactonisable.

Ces réactions peuvent être réalisées avec ou sans solvant. Comme solvant convenant à l'obtention de toluylène diisocyanate biurétisé, on peut citer par exemple, les composés choisis parmi les cétones telles que l'acétone, la méthyl éthyl cétone.

Le toluylène diisocyanate biurétisé ainsi obtenu, le cas échéant après élimination du solvant, se présente habituellement sous forme liquide.

Selon une autre caractéristique de l'invention, le toluylène diisocyanate modifié chimiquement n'est modifié que partiellement. En effet, si, dans le cas particulier de la préparation de mousses souples de polyuréthanne, le toluylène diisocyanate utilisé présente un taux de modification trop faible, la tenue du gel au moussage sera insuffisante. Par ailleurs, l'élasticité de la mousse obtenue pourra être jugée trop faible pour l'application. A l'inverse, si le taux de modification est trop élevé, la mousse obtenue risquera d'être fissurée du fait d'une gélification trop rapide par rapport à la formation de dioxyde de carbone. Il est donc nécessaire d'ajuster le taux de modification du toluylène diisocyanate selon l'application future de la mousse. A ce propos, on peut noter qu'il est à la portée de l'homme du métier de faire cet ajustement en contrôlant par exemple, directement et d'une manière classique, la viscosité du produit. Ainsi, dans le cas de réactions catalysées, on peut contrôler le degré de transformation en introduisant notamment dans le milieu réactionnel un agent désactivant le catalyseur.

Ainsi, selon un mode particulier, le mélange selon l'invention comprend un toluylène diisocyanate modifié chimiquement dont la teneur en liaisons - N=C=O est comprise entre 60 et 94 % par rapport à celle du toluylène diisocyanate de départ (soit une teneur de 6 à 40 % plus basse que celle du toluylène diisocyanate de départ). De préférence, la teneur en liaisons -N=C=O est comprise entre 80 et 90 % par rapport à celle du toluylène diisocyanate de départ (soit une teneur de 10 à 20 % plus basse que celle du toluylène diisocyanate de départ) et plus particulièrement, celle-ci est comprise entre 83 et 85 % par rapport à celle du toluylène diisocyanate de départ (soit une teneur de 15 à 17 % plus basse que celle du toluylène diisocyanate de départ).

Le mélange selon l'invention comprend par ailleurs du 1,6-hexaméthylène diisocyanate. Celui-ci peut être utilisé soit sous la forme de monomère purifié, soit sous la forme de produit brut. Par 1,6-hexaméthylène diisocyanate brut, on entend le 1,6-hexaméthylène diisocyanate en mélange avec ses produits de polymérisation ; ces derniers présentant des degrés de polymérisation plus ou moins élevés. Sous la dénomination de produits de polymérisation, on entend plus précisément les dimère, trimère, biuret et imino-urétidinone du 1,6-hexaméthylène diisocyanate.

Selon un mode particulier de réalisation de l'invention, le 1,6-hexaméthylène diisocyanate sous forme brute présente une teneur en produits de polymérisation comprise entre 17 et 88% en poids de 1,6-hexaméthylène diisocyanate brut. De préférence ladite teneur est inférieure à 50% en poids de 1,6-hexaméthylène diisocyanate.

Dans le cas où l'on utilise le 1,6-hexaméthylène diisocyanate sous la forme d'un monomère purifié, celui-ci présente plus particulièrement une pureté supérieure à 99,5 %. Selon un mode préféré de réalisation de l'invention, la pureté du monomère est supérieure à 99,8 %.

Le 1,6-hexaméthylène diisocyanate monomère présente l'avantage d'une plus grande simplicité de mise en oeuvre. En effet, il n'est pas nécessaire de filtrer le monomère, préalablement à son utilisation, puisque celui-ci ne contient pas de composés insolubles, alors que cela peut être le cas lors d'une mise en oeuvre de l'invention avec un mélange comprenant du 1,6-hexaméthylène diisocyanate brut. De plus, la faible acidité du 1,6-hexaméthylène diisocyanate monomère ne modifie pas globalement l'acidité du mélange et par conséquent ne change pas la cinétique de moussage.

Ainsi qu'il a été dit précedemment, le mélange selon l'invention comprend du toluylène diisocyanate modifié chimiquement et du 1,6-hexaméthylène diisocyanate ; ce dernier composé étant présent dans une quantité comprise entre 0,5 et 30 % en poids dudit mélange d'isocyanates.

De préférence, la quantité du 1,6-hexaméthylène diisocyanate varie entre 1 et 20 % en poids du mélange précité. Plus particulièrement, celle-ci varie entre 2,5 et 10 % en poids dudit mélange.

Avantageusement, les deux produits précités sont mis en contact par simple mélange, en présence ou non d'un solvant.

L'utilisation du mélange selon l'invention va maintenant être décrite.

L'obtention de mousses de polyuréthanne de haute élasticité à partir du mélange précité est réalisée en effectuant une réaction de polyaddition des isocyanates avec au moins un composé du type polyéther-polyol.

Ainsi qu'il a été dit auparavant, l'utilisation d'agent d'expansion physique pour effectuer la réaction n'est pas obligatoire. Dans le cas où un tel agent est cependant utilisé, ce dernier est choisi parmi les agents connus de l'homme du métier, et plus particulièrement parmi les agents qui ne sont pas concernés par le protocole de Montréal, comme le sont par exemple les chlorofluorocarbones.

Mais selon un mode préféré de l'invention, on effectue la réaction en présence d'un agent d'expansion chimique et plus particulièrement en présence d'eau.

La réaction de polyaddition peut être mise en oeuvre avec n'importe quel type de polyéther-polyol connu de l'homme du métier. Selon un mode particulier de réalisation de l'invention, les polyéthers-polyols utilisés présentent une masse moléculaire moyenne en nombre comprise entre 4800 et 6500.

Bien entendu, des additifs supplémentaires peuvent être utilisés, tels que notamment des catalyseurs, comme les amine tertiaires, des tensio-actifs comme les silicones ou d'autres composés silylés, des rétifiants comme la glycérine.

Les exemples suivants illustrent l'invention sans en limiter sa portée.

## EXEMPLES

Dans ce qui va suivre :

- TDI représente le 2,4-toluylène diisocyanate.
- HDI représente le 1,6-hexaméthylène diisocyanate.
- NCO représente la liaison isocyanate -N=C=O et OH la liaison hydroxyl - OH.
- p représente des parties en poids
- L'index I correspond à la formule suivante :

$$I = \frac{\text{nombre de groupements de NCO introduits}}{\text{nombre de groupements OH introduits}} \times 100$$

- Le protocole de mesure de la déformation conformément à la norme NF-T56-112 est le suivant :

Les éprouvettes à tester sont sous forme de parallélépipèdes de 50 mm de hauteur, à base carrée de 100 mm de côté.

La mesure de la hauteur initiale (Do) est faite sous une pression de 0,1 kPa (charge de 1 Newton) à l'aide d'un dynamomètre travaillant en compression.

Les échantillons sont mis en compression en étuve dans les conditions suivantes :

- taux de compression : 70 %
- atmosphère de référence : EH 95 % humidité relative

EP 0 600 774 B1

- température :                    50°C
- durée :                         22 heures

Dès la sortie de l'étuve, on libère l'éprouvette du dispositif d'essai.

On laisse reposer les éprouvettes à 23°C, EH 50 % pendant 30 minutes.

On mesure l'épaisseur résiduelle (Df) des éprouvettes dans les conditions de précharge décrites ci-dessus.

La déformation rémanente (D) après compression à déformation constante, exprimée en pourcentage, est donnée par la formule suivante dans laquelle (Dc) est l'épaisseur sous compression :

$$D = \frac{Do\text{-}Df}{Do\text{-}Dc} \times 100$$

## Exemples 1 à 5 :

On réalise un mélange polyol ayant la composition suivante:

| | |
|---|---|
| - ARCOL POLYOL 1372™ (société ARCO) | 100 p |
| - Eau | 3,1 p |
| - Glycérine | 0,8 p |
| - Agent tensio-actif: TEGOSTAB B4113® (société GOLDSCHMIDT) | 0,5 p |
| - 1,4 - Diazabicyclo (2,2,2) octane en solution à 33% (société Air Product) | 0,5 p |
| - Diméthylaminopropylamine | 0,4 p |
| - ARCOL POLYOL 1180™ (société ARCO) | 0,6 p |
| - Trichloroéthyl phosphate | 2 p |

On réalise les mélanges polisocyanates suivants:

| Mélanges polyisocyanates | TDI biurétisé nb.NCO/100g = 0,96 | HDI monomère nb.NCO/100g = 1,19 | nb.NCO dans 100g du mélange |
|---|---|---|---|
| Ex 1 | 97,5 % | 2,5 % | 0,966 |
| Ex 2 | 95 % | 5 % | 0,971 |
| Ex 3 | 92,5 % | 7,5 % | 0,977 |
| Ex 4 | 90 % | 10 % | 0,983 |
| Ex 5 comp. | 100 % | 0 % | 0,960 |

Les pourcentages dans ce tableau sont exprimés en poids par rapport à la quantité totale de TDI et de HDI présents dans ledit mélange.

Chacun de ces mélanges est ajouté à la composition polyol décrite ci-dessus de telle façon que la quantité du mélange ajouté permette d'obtenir des mousses dont l'index I est de 104,2.

On réalise 20 litres de mousse à partir de la masse totale des réactifs dans un expansomètre cylindrique thermostaté à 23°C à la base duquel se trouve un capteur de pression permettant l'enregistrement de la pression pendant le moussage (mesure de la durée d'initiation, de la durée au pic et de la pression maximum au pic).

Après 72 heures de stockage à pression atmosphérique à 23°C, en atmosphère à 50% d'humidité relative (EH), les blocs de mousse sont débités en parallélépipèdes de 50 mm de hauteur à base carrée de 100 mm de coté.

Le tableau 1 récapitule les caractéristiques de moussage, la densité des mousses obtenues, les résultats des mesures de déformation rémanente effectuées sur ces mousses, selon le protocole donné plus haut :

Tableau 1

| CARACTERISTIQUES | Ex 1 | Ex 2 | Ex 3 | Ex 4 | Ex 5 comp. |
|---|---|---|---|---|---|
| Durée initiation (s) | 16 | 16,5 | 17 | 19 | 16 |
| Durée au pic (s) | 91 | 85 | 88 | 91 | 88 |
| Pression au pic (hPa) | 6,4 | 8,2 | 6,5 | 7,1 | 7 |
| Densité à coeur (kg/m$^3$) | 34,6 | 36,9 | 35,9 | 38,4 | 34,5 |
| D (%) | 14 | 11 | 11 | 9 | 29 |

La même composition polyol est mise en oeuvre à l'index I de 90 avec les mélanges polyisocyanates décrits ci-dessus.

Les caractéristiques de moussage et les propriétés des mousses sont rassemblées dans le tableau 2 suivant :

Tableau 2

| CARACTERISTIQUES | Ex 1 | Ex 2 | Ex 3 | Ex 4 | Ex 5 comp. |
|---|---|---|---|---|---|
| Durée initiation (s) | 22 | 22 | 22 | 22 | 18 |
| Durée au pic (s) | 82 | 82 | 82 | 82 | 82 |
| Pression au pic (hPa) | 6,9 | 7,9 | 6,9 | 8 | 8,8 |
| Densité à coeur (kg/m$^3$) | 38 | 39,2 | 40 | 41,2 | 36,6 |
| D (%) | 14 | 14 | 9 | 9 | 50 |

La comparaison entre les tableaux 1 et 2 montre que la déformation rémanente des mousses selon l'invention n'est pas affectée par la modification de l'index I, alors qu'elle a doublé dans le cas des mousses classiques.

**Exemples 6 à 10 :**

Les mêmes mélanges polyisocyanates TDI et HDI monomère que ceux utilisés aux exemples 1 à 5 comparatif (respectivement correspondant aux exemples 6 à 10 comparatif) sont mis en oeuvre à l'index I de 104,2 avec une composition polyol identique à celle des exemples 1 à 5 comparatif, mais contenant 4,3 parties d'eau.

Les résultats obtenus sont rassemblés dans le tableau 3 suivant :

Tableau 3

| CARACTERISTIQUES | Ex 6 | Ex 7 | Ex 8 | Ex 9 | Ex 10 comp. |
|---|---|---|---|---|---|
| Durée d'initiation (s) | 22 | 22 | 25 | 25 | 22 |
| Durée au pic (s) | 100 | 106 | 112 | 109 | 106 |
| Pression au pic (hPa) | 7,2 | 8,6 | 10,3 | 9,3 | 8 |
| Densité à coeur (kg/m$^3$) | 27 | 27,8 | 27,1 | 29,3 | 25,7 |
| D (%) | 67 | 47 | 28 | 14 | 87 |

Le tableau ci-dessus montre que les mousses préparées selon l'invention présentent une déformation rémanente améliorée par rapport aux mousses classiques, même dans des conditions très défavorables, c'est-à-dire avec des teneurs en eau élevées.

### Exemples 11 à 15

Les mêmes mélanges polyisocyanates TDI et HDI que ceux utilisés aux exemples 1 à 5 comparatif sont mis en oeuvre à l'index I de 90 avec une composition de polyol identique à celle des exemples 1 à 5 comparatif mais contenant 4,3 parties d'eau.

Les résultats obtenus sont rassemblés dans le tableau 4 suivant :

Tableau 4

| CARACTERISTIQUES | Ex 11 | Ex 12 | Ex 13 | Ex 14 | Ex 15 comp. |
|---|---|---|---|---|---|
| Durée d'initiation (s) | 22 | 23 | 23 | 24 | 22 |
| Durée au pic (s) | 97 | 97 | 103 | 97 | 100 |
| Pression au pic (hPa) | 7,8 | 6,9 | 7,5 | 9,2 | 9,6 |
| Densité à coeur (kg/m$^3$) | 28,6 | 30,6 | 29,1 | 32,9 | 27,1 |
| D (%) | 69 | 50 | 35 | 19 | 94 |

La portance des mousses obtenues aux exemples 13 et 15 comparatif a été mesurée à divers degrés de compression.

Les résultats sont rassemblés dans le tableau 5 suivant :

Tableau 5

| Exemples | Portance (hPa) selon la compression (%) | | | |
|---|---|---|---|---|
| | 25 % | 40 % | 50 % | 65 % |
| 13 | 10,5 | 13,3 | 17,3 | 31,6 |
| 15 comparatif | 13,1 | 16,6 | 21,4 | 38,9 |

Ce tableau indique que les mousses selon l'invention ont une portance plus faible que les mousses classiques.

### Exemples 16 à 20

On réalise les mélanges de TDI biurétisé avec de l'HDI brut (contenant 53 % d'HDI libre et 47 % de lourds d'HDI) suivants :

| Mélange polyisocyanates | TDI biurétisé nb.NCO/100g=0,944 | HDI brut nb.NCO/100g=0,956 | nb.NCO/100 g du mélange |
|---|---|---|---|
| Ex 16 | 97,5 % | 2,5 % | 0,944 |
| Ex 17 | 95 % | 5 % | 0,944 |
| Ex 18 | 92,5 % | 7,5 % | 0,945 |
| Ex 19 | 90 % | 10 % | 0,945 |
| Ex 20 comparatif | 100 % | 0 % | 0,944 |

Ces mélanges isocyanates sont mis en oeuvre avec le mélange polyol utilisé aux exemples 6 à 10 comparatif (contenant 4,3 p d'eau) à l'index I de 104,3.

Les résultats obtenus sont rassemblés dans le tableau 6 suivant :

Tableau 6

| CARACTERISTIQUES | Ex 16 | Ex 17 | Ex 18 | Ex 19 | Ex 20 comp. |
|---|---|---|---|---|---|
| Durée d'initiation (s) | 19 | 25 | 24 | 25 | 19 |
| Durée au pic (s) | 91 | 97 | 94 | 103 | 91 |
| Pression au pic (hPa) | 6,3 | 5,8 | 5,9 | 5,1 | 8,6 |
| Densité à coeur (kg/m$^3$) | 29,2 | 29,9 | 30,4 | 30,4 | 27,2 |
| D (%) | 41 | 19 | 14 | 11 | 76 |

Ces mélanges de polyisocyanates sont mis en oeuvre avec le mélange polyol utilisé aux exemples 6 à 15 comparatif (contenant 4,3p d'eau) à l'index I de 90.

Les résultats obtenus sont rassemblés dans le tableau 7 suivant :

Tableau 7

| CARACTERISTIQUES | Ex 16 | Ex 17 | Ex 18 | Ex 19 | Ex 20 comp. |
|---|---|---|---|---|---|
| Durée d'initiation (s) | 22 | 22 | 22 | 22 | 22 |
| Durée au pic (s) | 88 | 88 | 91 | 97 | 85 |
| Pression au pic (hPa) | 7,1 | 6,8 | 5,8 | 4 | 11 |
| Densité à coeur (kg/m$^3$) | 29,8 | 30,8 | 31,9 | 32,9 | 28,9 |
| D (%) | 39 | 17 | 11 | 8 | 78 |

**Revendications**

1. Mélange de polyisocyanates caractérisé en ce qu'il comprend du toluylène diisocyanate modifié chimiquement et du 1,6-hexaméthylène diisocyanate dont la quantité est comprise entre 0,5 et 30 % en poids du mélange d'isocyanates.

2. Mélange selon la revendication 1 caractérisé en ce que la quantité du 1,6-hexaméthylène diisocyanate est comprise entre 1 et 20 % et, de préférence, entre 2,5 et 10 % en poids du mélange d'isocyanates.

3. Mélange selon la revendication 1 ou 2 caractérisé en ce que le toluylène diisocyanate modifié chimiquement correspond à des dérivés du toluylène diisocyanate comprenant des liaisons du type allophanate, biuret, uréthanne, urée, carbodiimide ou isocyanurate.

4. Mélange selon la revendication précédente caractérisé en ce que le toluylène diisocyanate modifié chimiquement est le toluylène diisocyanate comprenant des liaisons de type biuret.

5. Mélange selon l'une quelconque des revendications précédentes, caractérisé en ce que le toluylène diisocyanate modifié chimiquement possède une teneur en liaisons N=C=O comprise entre 60 et 94 % par rapport à celle du toluylène diisocyanate non modifié, plus particulièrement ladite teneur est comprise entre 80 et 90 %, et de préférence, celle-ci est comprise entre 83 et 85 %.

6. Mélange selon l'une quelconque des revendications précédentes, caractérisé en ce que le 1,6-hexaméthylène diisocyanate est soit sous forme purifiée, soit sous forme brute.

7. Mélange selon la revendication 6, caractérisé en ce que le 1,6-hexaméthylène diisocyanate sous forme purifiée présente une pureté supérieure à 99,5% et de préférence supérieure à 99,8%.

8. Mélange selon la revendication 6, caractérisé en ce que le 1,6-hexaméthylène diisocyanate sous forme brute présente une teneur en produits de polymérisation comprise entre 17 et 88% en poids de 1,6-hexaméthylène diisocyanate brut et de préférence ladite teneur est inférieure à 50% en poids de 1,6-hexaméthylène diisocyanate.

9. Utilisation du mélange selon l'une quelconque des revendications 1 à 8 pour la préparation de mousses souples de polyuréthanne de haute élasticité caractérisée en ce que l'on met en oeuvre le procédé de polyaddition en présence d'au moins un polyéther-polyol.

10. Utilisation selon la revendication 9, caractérisée en ce que ledit polyéther-polyol présente une masse moléculaire en nombre comprise entre 4800 et 6500.

11. Utilisation selon l'une des revendications 9 ou 10, caractérisée en ce que l'on met en oeuvre le procédé de polyaddition en présence d'un agent d'expansion chimique et de préférence en présence d'eau.

## Claims

1. A mixture of polyisocyanates, characterized in that it comprises chemically modified tolylene diisocyanate and 1,6-hexamethylene diisocyanate, the quantity of the latter being in the range 0.5% to 30% by weight of the mixture of isocyanates.

2. A mixture according to claim 1, characterized in that the quantity of 1,6-hexamethylene diisocyanate is in the range 1% to 20%, preferably in the range 2.5% to 10% by weight of the mixture of isocyanates.

3. A mixture according to claim 1 or claim 2, characterized in that the chemically modified tolylene diisocyanate is a tolylene diisocyanate derivative containing allophanate, biuret, urethane, urea, carbodiimide or isocyanurate type bonds.

4. A mixture according to the preceding claim, characterized in that the chemically modified tolylene diisocyanate is tolylene diisocyanate containing biuret type bonds.

5. A mixture according to any one of the preceding claims, characterized in that the chemically modified tolylene diisocyanate has a N=C=O bond content which is in the range 60% to 94% with respect to that of unmodified tolylene diisocyanate, more particularly said content is in the range 80% to 90%, preferably in the range 83% to 85%.

6. A mixture according to any one of the preceding claims, characterized in that the 1,6-hexamethylene diisocyanate is either in a purified or in an unrefined form.

7. A mixture according to claim 6, characterized in that the purified 1,6-hexamethylene diisocyanate is more than 99.5% pure, preferably more than 99.8% pure.

8. A mixture according to claim 6, characterized in that the unrefined 1,6-hexamethylene diisocyanate has a polymerisation products content which is in the range 17% to 88% by weight of unrefined 1,6-hexamethylene diisocyanate and preferably, said content is below 50% by weight of 1,6-hexamethylene diisocyanate.

9. The use of a mixture according to any one of claims 1 to 8 for the preparation of high elasticity flexible polyurethane foams, characterized in that the polyaddition process is carried out in the presence of at least one polyether-polyol.

10. Use according to claim 9, characterized in that said polyether-polyol has a number average molecular mass which is in the range 4800 to 6500.

11. Use according to claim 9 or claim 10, characterized in that the polyaddition process is carried out in the presence of a chemical blowing agent, preferably in the presence of water.

## Patentansprüche

1. Polyisocyanatmischung, dadurch gekennzeichnet, daß sie ein chemisch modifiziertes Toluylendiisocyanat und ein 1,6-Hexamethylendiisocyanat in einer Menge von zwischen 0,5 und 30 Gew.-% der Isocyanatmischung umfaßt.

2. Mischung nach Anspruch 1, dadurch gekennzeichnet, daß die Menge des 1,6-Hexamethylendiisocyanats zwischen 1 und 20 Gew.-% und vorzugsweise zwischen 2,5 und 10 Gew.-% der Isocyanatmischung umfaßt.

3.  Mischung nach Anspruch 1 oder 2, dadurch gekennzeichnet, daß das chemisch modifizierte Toluylendiisocyanat Derivaten des Toluylendiisocyanats entspricht, die Verbindungen des Typs Allophanat, Biuret, Urethan, Harnstoff, Carbodiimid oder Isocyanurat umfassen.

4.  Mischung nach einem der vorstehenden Ansprüche, dadurch gekennzeichnet, daß das chemisch modifizierte Toluylendiisocyanat das Toluylendiisocyanat ist, das Verbindungen des Typs Biuret umfaßt.

5.  Mischung nach einem der vorstehenden Ansprüche, dadurch gekennzeichnet, daß das chemisch modifizierte Toluylendiisocyanat einen Gehalt an Bindungen N=C=O besitzt, der im Bereich zwischen 60 und 94 % im Verhältnis zu demjenigen des nicht-modifizierten Toluylendiisocyanats liegt, insbesondere zwischen 80 und 90 % und vorzugsweise zwischen 83 und 85 %.

6.  Mischung nach einem der vorstehenden Ansprüche, dadurch gekennzeichnet, daß das 1,6-Hexamethylendiisocyanat entweder in gereinigter oder roher Form vorliegt.

7.  Mischung nach Anspruch 6, dadurch gekennzeichnet, daß das in gereinigter Form vorliegende 1,6-Hexamethylendiisocyanat eine Reinheit von großer als 99,5 % hat und vorzugsweise größer als 99,8 %.

8.  Mischung nach Anspruch 6, dadurch gekennzeichnet, daß das roh vorliegende 1,6-Hexamethylendiisocyanat einen Gehalt an Polymerisationsprodukten von 17 bis 88 Gew.-% des rohen 1,6-Hexamethylendiisocyanats umfaßt und vorzugsweise ist dieser Gehalt weniger als 50 Gew.-% des 1,6-Hexamethylendiisocyanats.

9.  Verwendung der Mischung nach einem der Ansprüche 1 bis 8 zur Herstellung eines Polyurethanweichschaums von hoher Elastizität, dadurch gekennzeichnet, daß man ein Polyadditionsverfahren in Gegenwart mindestens eines Polyetherpolyols einsetzt.

10. Verwendung nach Anspruch 9, dadurch gekennzeichnet, daß das Polyetherpolyol ein Zahlenmittel-Molekulargewicht von 4800 bis 6500 hat.

11. Verwendung nach Anspruch 9 oder 10, dadurch gekennzeichnet, daß man das Polyadditionsverfahren in Gegenwart eines chemischen Treibmittels und vorzugsweise in Gegenwart von Wasser durchführt.